# EUROPÄISCHE PATENTSCHRIFT

(11) **EP 2 258 339 B1**
(45) Veröffentlichungstag und Bekanntmachung des Hinweises auf die Patenterteilung: **07.11.2018**
(21) Anmeldenummer: 10401073.1
(22) Anmeldetag: 02.06.2010
(51) Int. Cl.: A61K 8/20, A61K 8/19, A61Q 11/00, A61K 8/26, A61K 8/96

(54) **Zahnreinigungsmittel**
A tooth-cleaning formulation
Produit de nettoyage pour dents

(30) Priorität: 02.06.2009 DE 202009007704 U
(43) Veröffentlichungstag der Anmeldung: 08.12.2010
(73) Patentinhaber: Hurtig, Sven, 09116 Chemnitz (DE)
(72) Erfinder: Hurtig, Sven, 09116 Chemnitz (DE)
(74) Vertreter: Rumrich, Gabriele

(56) Entgegenhaltungen:
- JP-A- 2007 254 315
- KR-B1- 100 811 594
- US-A- 4 374 824
- DATABASE GNPD [Online] MINTEL; März 2005 (2005-03), "Medicated Dental Paste XT", XP002725759, Database accession no. 351140
- DATABASE GNPD [Online] MINTEL; Juli 2004 (2004-07), "Beauty White", XP002725760, Database accession no. 284525

## Beschreibung

Die Erfindung betrifft ein Zahnreinigungsmittel, welches eine hervorragende Reinigungswirkung besitzt und insgesamt zur Verbesserung der Mundhygiene beiträgt.

Aus DE 3889895T2 ist eine Zahnpasta mit Tonerde bekannt, die aus einer homogenen Mischung aus Tonerde, Glycerin und Wasser besteht und die außerdem Meersalz enthalten kann. Ein Zahnreinigungsmittel unter Verwendung von Calciumsalz ist ebenfalls aus DE 699 35411 T2 bekannt.

In EP 0 297 563 B1 wird eine wässrige Zahnpastaformulierung, die ein Poliermittel, eine Quelle für lösliche Fluoridionen und einen Zink-ausgetauschten Zeolith als Zinkionenquelle enthält, bekannt, wobei diese aktiv ist bei der Inhibierung der Bildung von Zahnbelag und Zahnstein. Dabei erfolgt die Verwendung eines Zink-ausgetauschten Zeoliths mit einem Si0₂/Al₂0₃-Molverhältnis von 2,5 bis 10 als wenigstens 10 Gew.-% des Poliermittels und das Einstellen des pH-Wertes der Zusammensetzung auf über 9,5.

In KR 20 080 14 399 A wird darauf abgestellt, dass Himalaya Salz ausschließlich in einer hochprozessierten Form mit langzeitigem Glühen / Schmelzen (1200 °C bis 1300 °C), Abkühlen, Mahlen, Sterilisieren einen Nutzeffekt in Zahncreme aufweist. Hier stehen der Aufwand an Ressourcen, Energie wie auch Manpower in einem wenig nachvollziehbaren Verhältnis zu einem Nutzeffekt. Eine traditionelle Form dieses industriellen Verfahrens ist die seit hunderten Jahren bekannte Herstellung von "Bambussalz" bzw. jede Art einer alchemischen Zubereitung mineralischer Produkte. Wozu das hocherhitzte Salz noch zusätzlich sterilisiert werden muss, ist wenig nachvollziehbar.

Die DE 100 65 413 A1 definiert, dass ein gutes Zahnpflegemittel nur und genau mit Süßstoffen und Polyethylenglykol und Konservierungsmittel und technischen Fluorverbindungen und Farbstoffen und Pigmenten und Coenzym Q₁₀ oder / und Ubichinon (-derivate) oder / und Vitamin A auskommt.

In EP 1 965 765 B1 wird ein Zahnpflegemittel aufgezeigt, dass Siliciumdioxid-Nanopartikel besonders in einer "liposomalen Dispersion" unter weiterem Einsatz von "Glucanetherderivaten", enthält. Für diese speziellen Prozessprodukte werden sehr umfangreiche, energie-, ressourcen-, wie humankapital-intensive Verfahren als notwendig beschrieben.

Die Verwendung der vorgenannten Zahnreinigungsmittel ist möglich, jedoch unter ökologischer wie ökonomischer und technischer Betrachtung nicht optimal.

Aufgabe der vorliegenden Erfindung ist es, ein Zahnreinigungsmittel zu entwickeln, welches eine gute Reinigungswirkung aufweist, ohne den Zahnschmelz zu schädigen, vor Karies und Parodontose schützt und den Zahnschmelz revitalisieren kann. Der erfinderische Schritt liegt insbesondere darin, dass die Bestandteile weitestgehend in ihrer natürlichen Qualität, aus natürlichen Vorkommen und möglichst geringer technischer Verarbeitung den erfindungsgemäßen Zweck erfüllen, als auch alle Anforderungen an ein verkehrsfähiges Produkt erfüllt werden. Ressourcen- wie Energiesparende Verarbeitung haben gegenüber Natur wie auch Mensch oberste Priorität.

Diese Aufgabe wird mit den Merkmalen des ersten Patentanspruchs gelöst. Vorteilhafte Ausgestaltungen ergeben sich aus den Unteransprüchen.

Das Zahnreinigungsmittel enthält erfindungsgemäß naturbelassenes Himalaya Salz, welches nicht wärmebehandelt ist, und / oder Sango Koralle. Das Himalaya Salz wird dabei direkt als gewonnener natürlicher Rohstoff verwendet und bedarf keiner weiteren industriellen Behandlung. Es wird selbst nicht erhitzt, nicht chemisch gereinigt und nicht sterilisiert.

Dem erfindungsgemäßen Zahnreinigungsmittel kann zusätzlich Zeolith und / oder Bergkristall und / oder eine organische Siliciumquelle aus Schachtelhalm, als auch pflanzliche Zusätze (Kräuter) wie Salbei, Rosmarin, Teebaum, Nelke, Fenchel, Zimt, Minze und Minzearten, Eukalyptus, Kamille, Schachtelhalm, Neem, Echinacea, Anis, Myrrhe, Thymian, Zitrone einzeln oder in Kombinationen, als auch und schäumungserzeugende Mittel (Tenside) beigemengt sein.

Die Bestandteile Himalaya Salz, Sango Koralle wie auch Zeolith, Bergkristall, organische Siliciumquelle, können dabei fein, insbesondere mikrofein gemahlen sein.

Weiterhin kann das Zahnreinigungsmittel Kieselsäure und Kreide und / oder Bicarbonat (Natriumhydrogenkarbonat, Natron) enthalten. Außerdem kann in dem Zahnreinigungsmittel Glycerin und / oder Sorbitol und / oder Xylitol und / oder Mannit zum Einsatz kommen.

Im erfindungsgemäßen Zahnreinigungsmittel kann wie in der Zahnpflege üblich eine oder mehrere Fluoridquellen wie z.B. Natriumfluorid, Natriummonofluorphosphat oder Kaliumfluorid enthalten. Vorzugsweise wird jedoch auf natürliche Fluoridquellen wie z.B. Flussspat (Calciumfluorid / CaF₂) zurückgegriffen.

Des Weiteren kann das erfindungsgemäße Zahnreinigungsmittel eine oder mehrere Phosphatquellen, vorzugsweise Alkalimetallphosphate wie z.B. Natriumpolyphosphat enthalten.

Das erfindungsgemäße Zahnreinigungsmittel kann in Form von Zahnsalz, Zahnpulver oder Zahncreme, Zahnpaste, Zahngel oder Zahnpflegekaugummi oder Mundspüllösung vorliegen.

Das Zahnreinigungsmittel in Form von Zahnsalz bzw. Zahnpulver besteht dabei aus Himalaya Salz und / oder Sango Koralle, welches vorzugsweise mit Kräutern und / oder weiteren Putzkörpern wie Kieselerde/Silica, Kaolin, Natriumpolyphosphat, Zeolithe, Bergkristall, Kreide, Bicarbonat als auch Mineralerde versetzt wurde. Zahncreme bzw. Zahngel ist insbesondere eine wässrige Mischung (Emulsion) aus

| | |
|---|---|
| 0,9 - 60 % | Wasser und / oder Himalaya Salzsole |
| 1,0 - 40 % | Kieselsäure |
| 0,1 - 50 % | Kreide und / oder Bicarbonat |
| 1,0 - 60 % | Glycerin und / oder Sorbitol und / oder Xylitol und / oder Mannit |
| 0,1 - 5 % | Xanthan und / oder Alginat |
| 0,1 - 40 % | Himalaya Salz und / oder Sango Koralle, |

der vorzugsweise ebenfalls verschiedene Kräuter und schäumungserzeugende Mittel (Tenside) wie z.B. Sodium Cocoyl Glutamate und Disodium Cocoyl Glutamate (Kokostenside) oder Lauryl Glucoside (Zuckertensid) beigemengt sein können. Zusätzlich kann die Grundmischung Zeolithe (z.B. 0,1 bis 40%) und / oder Bergkristall (z.B. 0,1 bis 40 %) und / oder eine organische Siliciumquelle (z.B. 0,1 bis 40 %) enthalten.

Die hier aufgeführten Komponenten werden zu einer Zahncreme oder Zahngel zusammengeführt, in dem man die pulverförmigen Bestandteile inklusive naturbelassenes Himalaya Salz und / oder Sango Koralle langsam mischt. Anschließend lässt man die gewünschte Komposition der Feuchteträger einströmen und schlämmt damit die vorhandene Rezeptur auf. Damit wird die weitere sichere Verarbeitbarkeit gewährleistet (Staubfreiheit, Transportierbarkeit). In einem weiteren Prozessschritt wird Wasser und / oder Himalaya Salzsole zugefügt, als auch die Kräuter- und Geschmacksträgermischung. Nun findet eine abschließende Homogenisierung statt.

Auch eine andere Vorgehensweise ist möglich: Man mischt zuerst alle flüssigen Komponenten zusammen und gibt anschließend die pulverförmigen Komponenten inklusive naturbelassenes Himalaya Salz und / oder Sango Koralle hinzu. Zum Schluss werden die Geschmacksträger beigemischt.
Der Mischvorgang in beiden Varianten kann bei Bedarf jeweils in Vakuum erfolgen.

Wird das Zahnreinigungsmittel in Form von Mundspüllösung hergestellt, ist dies vorzugsweise eine 0,9 bis 27%ige Solelösung aus Himalaya Salz bedarfsweise mit Zusätzen in Form von Auszügen aus verschiedenen Kräutern.

Der pH-Wert des Zahnreinigungsmittels kann bevorzugt 7 bis 8,5 betragen.

Die erfindungsgemäße Zahncreme reinigt die Zähne und kräftigt das Zahnfleisch insbesondere durch eine Kombination aus Himalaya Salz und unterschiedlichen Heilpflanzen. Mineralische Putzkörper aus Kreide, Bicarbonat und Salzkristallen entfernen Zahnbeläge äußerst schonend.

Himalaya Salz ist außerordentlich reich an Mineralien, wie Calcium, Magnesium, Natrium, Kalium, Sulfat und enthält darüber hinaus weitere Spurenelemente. Auch für die Zähne wichtiges Fluorid ist in natürlicher gebundener Form enthalten. Es wirkt antibakteriell, durchblutungsfördernd, regt den natürlichen Speichelfluss an, unterstützt die Selbstreinigung der Zähne und neutralisiert schädliche Säuren.

Das Calcium der Sango Koralle mineralisiert den Zahnschmelz. Die Sango Koralle - auch Sango Calcium genannt - stellt dabei einen natürlichen Mineralstoffkomplex zur Verfügung, denn sie enthält alle essentiellen Mineralstoffe und wirkt weiterhin einer Übersäuerung entgegen.

Zeolithe aus der Zeolithgruppe bildet eine artenreiche Familie chemisch recht komplexer Silikat-Minerale. Man hat festgestellt, dass sich insbesondere mikrofeines Zeolith als Putzkörper eignet, Schadstoffe und Schwermetalle binden und Mundgeruch neutralisieren kann.

Auszüge aus verschiedenen Kräutern in Form von Extrakten, Tinkturen oder natürlichen ätherischen Ölen stärken Zahnfleisch und Mundflora, wirken entzündungshemmend, antiseptisch und antibakteriell.

Die Erfindung wird nachfolgend anhand von Ausführungsbeispielen näher erläutert.

### 1. Beispiel:

Zur Herstellung von Zahncreme wird eine wässrige Mischung (Paste) aus 0,9 - 60% Wasser, 1 - 40 % Kieselsäure, 0,1 - 50 % Kreide und / oder Bicarbonat, 1 - 60 % Glycerin und / oder Sorbitol und / oder Xylitol und / oder Mannit und 0,1 - 5 % Xanthan und / oder Alginat sowie 0,1 - 40 % Himalaya Salz und / oder Sango Koralle hergestellt. Dieser Grundsubstanz wurde Salbei, Rosmarien und Teebaum sowie Schachtelhalm, Neem, Thymian und Myrrhe beigemengt. Es wird damit eine Zahncreme geschaffen, die einen natürlichen Schutz vor Karies und Parodontose bildet und Zahnfleischbluten vorbeugt. Die Zahncreme wirkt antibakteriell, antiseptisch und entzündungshemmend und kann in der Zahnheilkunde zur Wundheilung eingesetzt werden. Das Teebaumöl beugt Entzündungen vor und ist besonders bei empfindlichem Zahnfleisch geeignet. Bedarfsweise kann zusätzlich eine Fluoridquelle eingesetzt werden.

### 2. Beispiel:

Der Grundsubstanz aus Beispiel 1 werden Auszüge der Kräuter Nelke, Fenchel und Zimt, ergänzt mit Schachtelhalm, Neem, Myrrhe und Echinacea beigefügt, wodurch die Zahncreme ebenfalls einen natürlichen Schutz vor Karies und Parodontose bildet. Diese Zahncreme wirkt ebenfalls antibakteriell, antiseptisch und entzündungshemmend. Die Anwendung von Nelken unterstützt die Schmerzlinderung und Echinacea die Abwehrfunktion der natürlichen Mundflora. Die mentholfreie Zahncreme ist bei homöopathischer Behandlung geeignet. Bedarfsweise kann zusätzlich eine Fluoridquelle eingesetzt werden.

### 3. Beispiel:

Der Grundmischung aus 0,9 - 60% Wasser und / oder Himalaya Salzsole , 1 - 40 % Kieselsäure, 0,1 - 50 % Kreide und / oder Bicarbonat, 1 - 60 % Glycerin und / oder Sorbitol und / oder Xylitol und / oder Mannit und 0,1 - 5 % Xanthan und / oder Alginat und 0,1 - 40 % Himalaya Salz und / oder Sango Koralle wird ein Kräuterauszug aus Minze und Minzearten, Eukalyptus und Kamille, ergänzt mit Schachtelhalm, Neem, Salbei und Myrrhe beigefügt. Bedarfsweise kann zusätzlich eine Phosphat- und / oder Fluoridquelle eingesetzt werden. Die Zahncreme wirkt ebenfalls antibakteriell, antiseptisch und entzündungshemmend. Kamille beugt Zahnfleischbluten vor und wird in der Zahnheilkunde zur Wundheilung eingesetzt. Es wird ein natürlicher Schutz vor Karies und Parodontose erhöht. Minze und Menthol sorgen für einen angenehmen Geschmack und frischen Atem.

### 4. Beispiel:

Der Grundmischung aus Beispiel 3 werden statt pulverigem Himalaya Salz nur Sango Koralle (0,1 - 40 %), Zeolithe (0,1 - 40 %), Bergkristall (0,1 - 40 %) beigefügt und bedarfsweise Kaliumchlorid (0,1 - 12 %), eine organische Siliciumquelle (0,1 - 40 %), eine weitere organische Calciumquelle (0,1 - 40 %), eine Phosphatquelle (0,1 - 12 %), eine Fluoridquelle (0,1 - 12 %) und statt Wasser nur Himalaya Salzsole (0,9 - 60 %).

Dieses neuartige Zahngel mineralisiert die Zähne und kräftigt das Zahnfleisch durch eine Kombination aus Himalaya Salzsole und z.B. natürlichen Mineralien. Putzkörper aus Kreide, Kieselerde und Zeolith entfernen Zahnbelege schonend. Kieselerde, auch Silicea genannt, stärkt das Bindegewebe und festigt die Zähne. Mikrofein gemahlenes Zeolith bindet Schadstoffe und Schwermetalle und neutralisiert Mundgeruch. Der Wirkstoff Kaliumchlorid dringt über Dentin-Kanälchen in den Zahn ein und bildet einen Schutzmantel um den Zahnnerv. Der Zahn wird beruhigt und Schmerzempfindlichkeit reduziert. Das Calcium der Sango Koralle und bedarfsweise weiterer organischer Calciumquellen, sowie eine Fluoridquelle, vorzugsweise natürliches Calciumfluorid (Flussspat) remineralisieren den Zahnschmelz. Zusätzliche, dem Zahngel beigefügte Auszüge aus Kräutern, wie aus Schachtelhalm, Neem, Echinacea, Salbei, Myrrhe, Anis, Krausminze (mentholfrei) und Zitrone und stärken Zahnfleisch und Mundflora, wirken entzündungshemmend, antiseptisch und antibakteriell. Das mikrofein gemahlene Bergkristallpulver und bedarfsweise eine organische Siliciumquelle vitalisiert und stärkt Zähne, Zahnfleisch und Mundflora. Dieses Zahngel ist ebenfalls bei regelmäßiger Anwendung ein natürlicher Schutz vor Karies und Parodontose. Das Zahngel in Beispiel 4 ist mentholfrei und bei homöopathischer Behandlung geeignet.

### 5. Beispiel:

Eine weitere Ausführungsform des erfindungsgemäßen Zahnreinigungsmittels stellt der folgende Zahnpflegekaugummi dar.

Die Kaumasse besteht dabei vorzüglich aus:

| | |
|---|---|
| 10 - 40 % | Gummigrundlage sog. "Gumbase" |
| 10 - 70 % | Xylitol und / oder Mannit |
| 1 - 50 % | Glycerin und / oder Sorbitol |
| 0,1 - 12 % | Himalaya Salz und / oder Sango Koralle |
| 0,1 - 5 % | Kräuterextrakte und / oder Gewürzextrakte |
| 0 - 3,2 % | organischer Verdicker |

Bedarfsweise kann der Grundmischung zusätzlich eine Phosphatquelle z.B. Natriumpolyphosphat und weitere Mineralträger und Putzkörper wie Kieselerde/Silica, Kaolin, Bergkristall, Kreide, Bicarbonat, eine organische Siliciumquelle als auch eine organische Calciumquelle beigemengt werden.

Hinweise:
- Himalaya Salz wird auch bezeichnet als Himalajasalz, Himalaya Kristallsalz, Himalaya Steinsalz, Kristallsalz vom Fuße des Himalaya, Salt Range Steinsalz, Kristall-Salz, Kristallsalz aus der Salt Range im Himalaya, Kristallsalz aus dem Salzgebirge, Indus Salz, Himalaya Indussalz, Indussalz aus dem Himalaya, Kaisersalz, Alexandersalz, Tibet Salz, Tibet Salt, Hunzasalz, Hunza-Kristallsalz, Hunza Salz, Christ-All-Salz, ayurvedisches Zaubersalz, Himalayan Rock Salt, Salt Range Rock Salt, Halit Crystal Salt, Himalaya Halit Salt, Crystal Salt Pink, Himalayan Pink Salt, Himalayan Crystal Salt, Indus Himalayasalz
- Die Sango Korallen unterteilten sich in die Sango Meereskorallen und die Sango Landkorallen, die beide für die erfindungsgemäße Anwendung geeignet sind.

Zusammenfassend kann festgestellt werden, dass es mit der vorliegenden Erfindung und Verfahrensweise gelungen ist, auf Ressourcen- und Energiesparende Art und Weise ein Zahnreinigungsmittel mit Sango Koralle und / oder Himalaya Salz herzustellen, ohne das Himalaya Salz auf sehr hohe Temperaturen erhitzen, zum Schmelzen bringen und Sterilisieren zu müssen, sondern es direkt als naturbelassenen Rohstoff zu verwenden. Die ökologischen wie ökonomischen Faktoren wurden mit dem hier beschriebnen erfindungsgemäßen Zahnreinigungsmittel gegenüber dem Nutzeffekt deutlich verbessert.

## Patentansprüche

1. Zahnreinigungsmittel, **dadurch gekennzeichnet, dass** es
- Himalaya Salz in naturbelassener, wie auch nicht wärmebehandelter Form und/oder Sango Koralle und
- Xylitol
enthält,
wobei das Himalaya Salz dabei als direkter natürlicher Rohstoff verwendet wird und selbst nicht erhitzt und nicht sterilisiert ist
und
dass das Zahnreinigungsmittel Zeolithe und/oder Bergkristall und/oder eine organische Siliciumquelle enthält, welche pulverförmig sind
und
dass das Zahnreinigungsmittel pflanzliche Zusätze in Form von Salbei, Rosmarin, Teebaum, Nelke, Fenchel, Zimt, Minze und Minzearten, Eukalyptus, Kamille, Schachtelhalm, Neem, Echinacea, Anis, Myrrhe, Thymian, Zitrone einzeln oder in Kombinationen enthält.

2. Zahnreinigungsmittel nach Anspruch 1, **dadurch gekennzeichnet, dass** es Kieselsäure, Kreide, Bicarbonat in Form von Natriumhydrogenkarbonat, Glycerin, Sorbitol, Mannit einzeln oder in Kombinationen enthält.

3. Zahnreinigungsmittel nach einem der Ansprüche 1 und 2, **dadurch gekennzeichnet, dass** es eine Phosphatquelle in Form von Natriumpolyphosphat, Dicalciumphosphat, eine natürliche Fluoridquelle in Form von Flussspat, eine in der Zahnpflege übliche Fluoridquelle in Form von Natriumfluorid, Natriummonofluorphosphat, Kaliumfluorid einzeln oder in Kombinationen enthält.

4. Zahnreinigungsmittel nach einem der Ansprüche 1 bis 3, **dadurch gekennzeichnet, dass** es in Form von Zahnsalz, Zahnpulver oder Zahncreme, Zahnpaste, Zahngel oder Zahnpflegekaugummi oder Mundspüllösung vorliegt.

5. Zahnreinigungsmittel nach einem der Ansprüche 1 bis 4, **dadurch gekennzeichnet, dass** das Zahnsalz bzw. Zahnpulver aus Himalaya Salz und / oder Sango Koralle bedarfsweise in Verbindung mit Kräutern weitere Putzkörpern in Form von Kieselerde, Kaolin, Natriumpolyphosphat, Zeolithe, Bergkristall, Kreide, Bicarbonat als auch Mineralerde aufweist.

6. Zahnreinigungsmittel nach einem der Ansprüche 1 bis 4, **dadurch gekennzeichnet, dass** die Zahncreme bzw. Zahngel eine Mischung aus
| | |
|---|---|
| 0,9 - 60 % | Wasser und / oder Himalaya Salzsole |
| 1,0 - 40 % | Kieselsäure |
| 0,1 - 50 % | Kreide und / oder Bicarbonat |
| 1,0 - 60 % | Glycerin und / oder Sorbitol und / oder Xylitol und / oder Mannitol |
| 0,1 - 5 % | Xanthan und / oder Alginat |
| 0,1 - 40 % | Himalaya Salz und / oder Sango Koralle |
ist, der bedarfsweise verschiedene Kräuter und schäumungserzeugende Mittel in Form von Sodium Cocoyl Glutamate und Disodium Cocoyl Glutamate oder Lauryl Glucoside beigemengt sein können.

7. Zahnreinigungsmittel nach Anspruch 6, **dadurch gekennzeichnet, dass** es 0,1 bis 40% Zeolith und / oder 0,1 bis 40 % Bergkristall und / oder 0,1 bis 40 % einer organischen Siliciumquelle enthält.

8. Zahnreinigungsmittel nach einem der Ansprüche 1 bis 4, **dadurch gekennzeichnet, dass** der Zahnpflegkaugummi aus
| | |
|---|---|
| 10 - 40 % | Gummigrundlage sog. "Gumbase" |
| 10 - 70 % | Xylitol und / oder Mannit |
| 1 - 50 % | Glycerin und / oder Sorbitol |
| 0,1 - 12 % | Himalaya Salz und / oder Sango Koralle |
| 0,1 - 5 % | Kräuterextrakte und / oder Gewürzextrakte |
| 0 - 3,2 % | organischer Verdicker |
besteht, der bedarfsweise zusätzlich eine Phosphatquelle in Form von Natriumpolyphosphat und weitere Mineralträger und Putzkörper in Form von Kieselerde, Kaolin, Bergkristall, Kreide, Bicarbonat, eine organische Siliciumquelle beigemengt sein können.

## Claims

1. Dental cleaning agent, **characterized in that** it contains
- Himalayan salt in natural and also non-heat-treated form and/or Sango coral
and
- xylitol, wherein the Himalayan salt is used in this case as a direct natural raw material and is itself not heated and not sterilized,
and
- that the dental cleaning agent contains zeolites and/or rock crystal
- and/or an organic silicon source which is powdered, and
that the dental cleaning agent contains plant additives in the form of sage, rosemary, tea tree, clove, fennel, cinnamon, mint and mint species, eucalyptus, camomile, horsetail, neem, echinacea, anise, myrrh, thyme, lemon individually or in combinations.

2. Dental cleaning agent according to claim 1, **characterized in that** it contains silica, chalk, bicarbonate in the form of sodium bicarbonate, glycerol, sorbitol, mannitol, individually or in combinations.

3. Dental cleaning agent according to one of claims 1 and 2, **characterized in that** it contains a source of phosphate in the form of sodium polyphosphate, dicalcium phosphate, a natural source of fluoride in the form of fluorspar, a source of fluoride customary in dental care in the form of sodium fluoride, sodium monofluorophosphate, potassium fluoride, individually or in combinations.

4. Dental cleaning agent according to one of claims 1 to 3, **characterized in that** it is in the form of tooth salt, tooth powder or dental cream, toothpaste, tooth gel or dental chewing gum or mouth rinse solution.

5. Dental cleaning agent according to one of claims 1 to 4, **characterized in that** the tooth salt or tooth powder made of Himalayan salt and/or Sango coral may comprise, in combination with herbs, further cleaning bodies in the form of silica, kaolin, sodium polyphosphate, zeolites, rock crystal, chalk, bicarbonate and mineral earth.

6. Dental cleaning agent according to one of claims 1 to 4, **characterized in that** the toothpaste or tooth gel contains a mixture of
0.9 - 60% water and/or Himalaya brine
1.0- 40% silica
0.1 - 50% chalk and/or bicarbonate
1.0 - 60% glycerol and/or sorbitol and/or xylitol and/or mannitol
0.1 - 5% xanthan and/or alginate
0.1 - 40% Himalayan salt and/or Sango coral,
which may be admixed as required with various herbs and foaming agents in the form of sodium cocoyl glutamate and disodium cocoyl glutamate or lauryl glucosides.

7. Dental cleaning agent according to claim 6, **characterized in that** it contains 0.1 up to 40% zeolite and/or 0.1 to 40% rock crystal and/or 0.1 to 40% of an organic silicon source.

8. Dental cleaning agent according to one of claims 1 to 4, **characterized in that** the dental chewing gum consists of
10 - 40% rubber base, so-called "gumbase"
10 - 70% xylitol and/or mannitol
1 - 50 % glycerol and/or sorbitol
0.1 - 12 % Himalayan salt and/or Sango coral
0.1 - 5% herbal and/or spice extracts
0 - 3.2% organic thickener
to which additionally a phosphate source in the form of sodium polyphosphate and other mineral carriers and cleaning bodies in the form of silica, kaolin, rock crystal, chalk, bicarbonate, an organic silicon source can be added as required.

## Revendications

1. Produit pour le nettoyage des dents, **caractérisé en ce qu'**il contient
- du sel de l'Himalaya sous sa forme naturelle et non traitée thermiquement et/ou du corail Sango
et
- du xylitol,
le sel de l'Himalaya étant utilisé directement comme matière première naturelle sans être chauffé ni stérilisé,
et
**en ce que** le produit pour le nettoyage des dents contient des zéolithes et/ou du cristal de roche et/ou une source de silicium organique en poudre
et
**en ce que** le produit pour le nettoyage des dents contient des additifs végétaux sous la forme de sauge, de romarin, d'arbre à thé, de girofle, de fenouil, de cannelle, de menthe et d'espèces de menthe, d'eucalyptus, de camomille, de prêle, de neem, d'échinacée, d'anis, de myrrhe, de thym, de citron, seuls ou en combinaisons.

2. Produit pour le nettoyage des dents selon la revendication 1, **caractérisé en ce qu'**il contient de l'acide silicique, de la craie, du bicarbonate sous forme d'hydrogénocarbonate de sodium, de la glycérine, du sorbitol, du mannite, seuls ou en combinaisons.

3. Produit pour le nettoyage des dents selon l'une des revendications 1 et 2, **caractérisé en ce qu'**il contient une source de phosphate sous la forme du polyphosphate de sodium, du phosphate dicalcique, une source naturelle de fluorure sous la forme de spath fluor, une source de fluorure usuelle en soin dentaire sous la forme du fluorure de sodium, du fluorophosphate de sodium, du fluorure de potassium, seuls ou en combinaisons.

4. Produit pour le nettoyage des dents selon l'une des revendications 1 à 3, **caractérisé en ce qu'**il se présente sous forme de sel dentaire, de poudre dentifrice ou de crème dentifrice, de pâte dentifrice, de gel dentifrice ou de gomme à mâcher pour soins dentaires ou de solution pour bains de bouche.

5. Produit pour le nettoyage des dents selon l'une des revendications 1 à 4, **caractérisé en ce que** le sel dentaire ou la poudre dentifrice composé(e) de sel de l'Himalaya et/ou de corail Sango, au besoin associés à des herbes, contient d'autres particules abrasives sous la forme de terre à diatomées, de kaolin, de polyphosphate de sodium, de zéolithes, de cristal de roche, de craie, de bicarbonate ou de terre minérale.

6. Produit pour le nettoyage des dents selon l'une des revendications 1 à 4, **caractérisé en ce que** la crème dentifrice ou le gel dentifrice contient un mélange
de 0,9 à 60 % d'eau ou de saumure de sel de l'Himalaya,
de 1,0 à 40 % d'acide silicique,
de 0,1 à 50 % de craie et/ou de bicarbonate,
de 1,0 à 60 % de glycérine et/ou de sorbitol et/ou de xylitol et/ou de mannitol,
de 0,1 à 5 % de gomme xanthane et/ou d'alginate,
de 0,1 à 40 % de sel de l'Himalaya et/ou de corail Sango,
auxquels peuvent être ajoutés, si besoin, différentes herbes et des agents moussants sous forme de cocoyl-glutamate de sodium et de cocoyl-glutamate disodique ou de lauryl-glucoside.

7. Produit pour le nettoyage des dents selon la revendication 6, **caractérisé en ce qu'**il contient entre 0,1 et 40% de zéolithe et/ou entre 0,1 et 40 % de cristal de roche et/ou entre 0,1 et 40 % d'une source de silicium organique.

8. Produit pour le nettoyage des dents selon l'une des revendications 1 à 4, **caractérisé en ce que** la gomme à mâcher pour le soin des dents se compose
de 10 à 40 % de gomme-base,
de 10 à 70 % de xylitol et/ou de mannite,
de 1 à 50 % de glycérine et/ou de sorbitol,
de 0,1 à 12 % de sel de l'Himalaya et/ou de corail Sango,
de 0,1 à 5 % d'extraits d'herbes et/ou d'extraits d'épices,
de 0 à 3,2 % d'épaississants organiques,
auxquels peuvent être ajoutés, si besoin, une source de phosphate sous la forme de polyphosphate de sodium et d'autres véhicules minéraux et agents abrasifs sous la forme de terre à diatomées, de kaolin, de cristal de roche, de craie, de bicarbonate, une source organique de silicium.
